Europäisches Patentamt

(19) **European Patent Office**

Office européen des brevets

(11) Publication number: **0 093 538**
**B1**

(12) **EUROPEAN PATENT SPECIFICATION**

(45) Date of publication of the patent specification:
**25.09.85**

(51) Int. Cl.⁴: **A 61 K 9/22,** A 61 K 33/00,
A 61 K 33/14

(21) Application number: **83302216.3**

(22) Date of filing: **19.04.83**

(54) Sustained release lithium-containing tablets and method for their manufacture.

(30) Priority: **30.04.82 GB 8212636**

(43) Date of publication of application:
**09.11.83 Bulletin 83/45**

(45) Publication of the grant of the patent:
**25.09.85 Bulletin 85/39**

(84) Designated Contracting States:
**AT BE CH DE LI NL**

(56) References cited:
**GB - A - 1 209 093**
**GB - A - 1 450 536**
**GB - A - 2 016 922**

**CHEMICAL ABSTRACTS, vol. 90, no. 6, 5th February 1979, pages 304-305, no. 43735u, Columbus, Ohio, USA K. VENTOURAS et al.: "Role of technological factors on the release from hydrophilic matrixes" K. VENTOURAS et al.,Expo. Congr. Int. Technol. Pharm. 1st 1977, (4), pp. 104-113**

**The file contains technical information submitted after the application was filed and not included in this specification**

(73) Proprietor: **DELANDALE LABORATORIES LIMITED, Delandale House 37 Old Dover Road, Canterbury Kent CT1 3JB (GB)**

(72) Inventor: **Trigger, David John, 32, Harkness Drive, Canterbury Kent (GB)**

(74) Representative: **Ellis, John Clifford Holgate et al, MEWBURN ELLIS & CO. 2/3 Cursitor Street, London EC4A 1BQ (GB)**

# Description

The sustained release lithium carbonat tablets manufactured by the applicant company have been well received by the medical profession.

Lithium salts are employed in the treatment of affective disorders and in particular as a prophylactic in recurrent manic-depressive or depressive illness. The virtue of the sustained release lithium carbonate tablets is that they release lithium at a predetermined rate over a fairly prolonged period, thus enabling the tablets to be taken less frequently than if the lithium was available immediately on consumption of the tablet while still providing an acceptable blood level.

A sustained release lithium carbonate tablet was described in GB-A No. 1209093, this tablet comprising—

(*i*) a powdered therapeutically active lithium salt;

(*ii*) a sustained release agent comprising a mixture of glyceryl mono-, di- and tri-esters of one or more straight chain $C_{16}$-$C_{18}$ saturated fatty acids, and

(*iii*) other innocuous compouding materials.

The tablets may be made by a method which comprises granulation of the above mixture by a moist granulation technique, screening to form a granulate of appropriate grain sizes and compacting the granulate into tablets. A process of this kind is also disclosed in GB-A No. 2016922. In addition, K. Ventouras *et al.* in Expo. Congr. Int. Technol. Pharm. 1$^{st}$ 1977 (4), pp. 104-113, have studied the sustained release of $LiSO_4$ crystals embedded in a hydrophilic matrix. According to this study no clear correlation can be made between the particle size and the releasing rate of $LiSO_4$ or of other lithium salts in such a matrix.

We have found that difficulties still remained in the manufacture of sustained release lithium carbonate tablets which would give a satisfactory release pattern of lithium over an extended period. In fact, batches of the tablets were found to give release times which exceeded the desired limits—in other words the release of lithium in the usual *in vitro* release pattern testing procedures was too slow and thus, had the tablets been ingested, the release of lithium would have been insufficient to provide a satisfactory therapeutic level.

After considerable investigation and experimentation the cause of the trouble was unexpectedly found to be in the nature of the crystals of lithium carbonate themselves.

The present invention thus relates to a sustained release lithium carbonate tablet containing particles of lithium carbonate coated with a sustained release agent characterized in that the lithium carbonate material comprises acicular crystals with an essentially monomodal particle size distribution in which the curve showing volume percentages of particles of different sizes shows a peak at a particle diameter of between 10 and 25 µm, not more than 20% by volume (preferably less than 10% by volume) of the material having a particle diameter above 30 µm.

A very suitable sustained release agent for coating the lithium carbonate is a mixture of glyceryl mono-, di- and tri-esters of one or more straight chain saturated fatty acids. Such a material is available and sold by Messrs. Gattefosse of France under the Trade Mark "Precirol", the acid radicals being principally the radicals of palmitic and stearic acids.

A method of making the tablets of the present invention comprises the steps of—

(*a*) mixing (*i*) acicular crystals of lithium carbonate having an essentially monomodal particle size distribution in which the curve showing volume percentages of particles of different sizes shows a peak at a particle diameter of between 10 and 25 µm, not more than 20% by volume of the material having a particle diameter above 30 µm; (*ii*) a sustained release agent comprising a mixture of glyceryl mono-, di- and tri-esters of one or more straight chain saturated fatty acids; and (*iii*) other innocuous compounding materials;

(*b*) granulation of the said mixture by a moist granulation technique;

(*c*) screening to form a granulate of appropriate grain sizes,

(*d*) compacting the granulate into tablets.

The said other innocuous compounding materials may include a filler such as mannitol, a binding agent such as gum acacia, and a lubricant such as magnesium stearate.

The aforementioned screening should preferably give a granulate whose particles all pass through a sieve with 1.40 mm apertures, where 20 to 30% pass through a sieve with 250 µm apertures while 70 to 80% are retained on a sieve with 250 µm apertures. A lubricant, such as magnesium stearate, may be added to the granulate after screening and before tabletting.

In an embodiment of the invention, not more than 10% of the lithium carbonate material has a particle diameter above 30 µm.

*Description of the preferred embodiments*

The invention will be further described with reference to the accompanying drawings, wherein:

Fig. 1 is a scanning electron micrograph of lithium carbonate material previously used and found to give batches of tablets with insufficiently controlled release times;

Fig. 2 is a particle size distribution analysis of the material whose electron micrograph is given in Fig. 1;

Fig. 3 is a scanning electron micrograph of lithium carbonate material found to provide satisfactory tablets, and

Fig. 4 is a particle size distribution analysis of the material whose electron micrograph is given in Fig. 3.

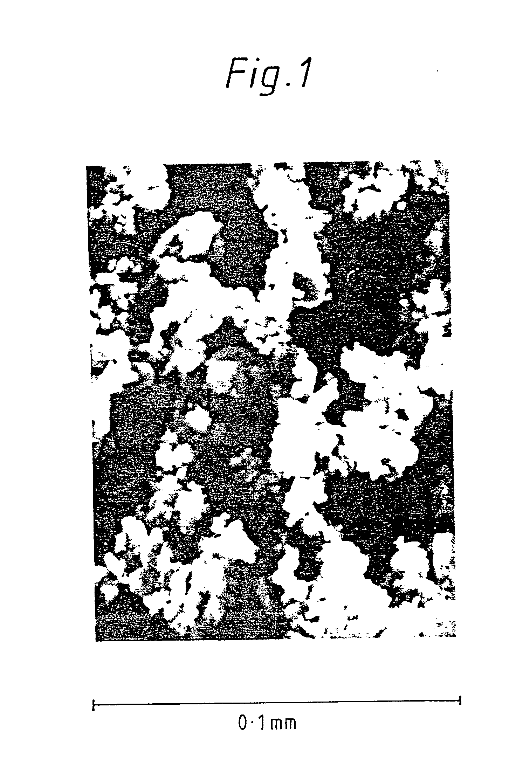

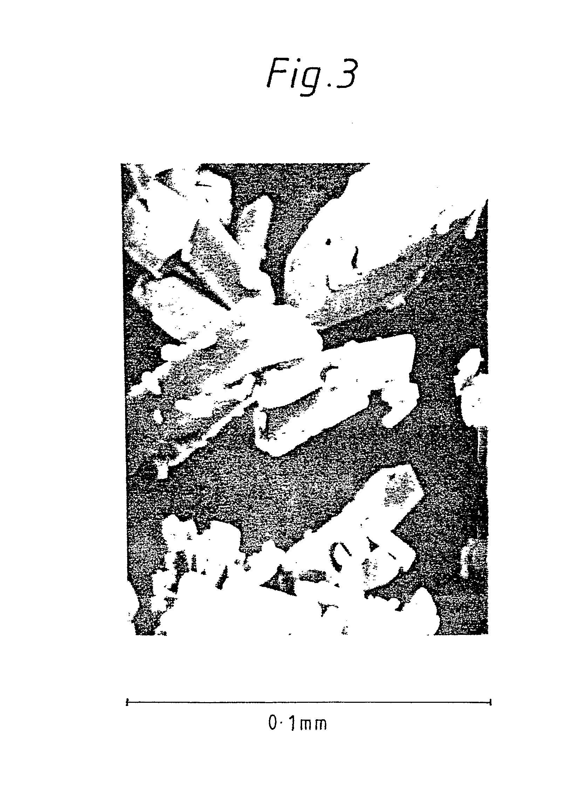

A comparison of Fig. 1 and 3 shows the essential difference between the crystalline lithium carbonate material found to be unsatisfactory, and that found to give reliably constant results. The lithium carbonate of Fig. 1 consists of minute crystals clustered together whilst the material of Fig. 3 has larger, essentially single acicular crystals. Naturally the particle sizes vary, but we have found that satisfactory results are achieved, as long as the curve showing volume percentages of particles of different sizes shows a peak at a particle diameter of between 10 and 25 μm and not more than 20% by volume of the material has a particle diameter above 30 μm. By "particle diameter" we mean the diameter of a circle having the same area as the area of the profile of the particle, as seen by the particle size analyser.

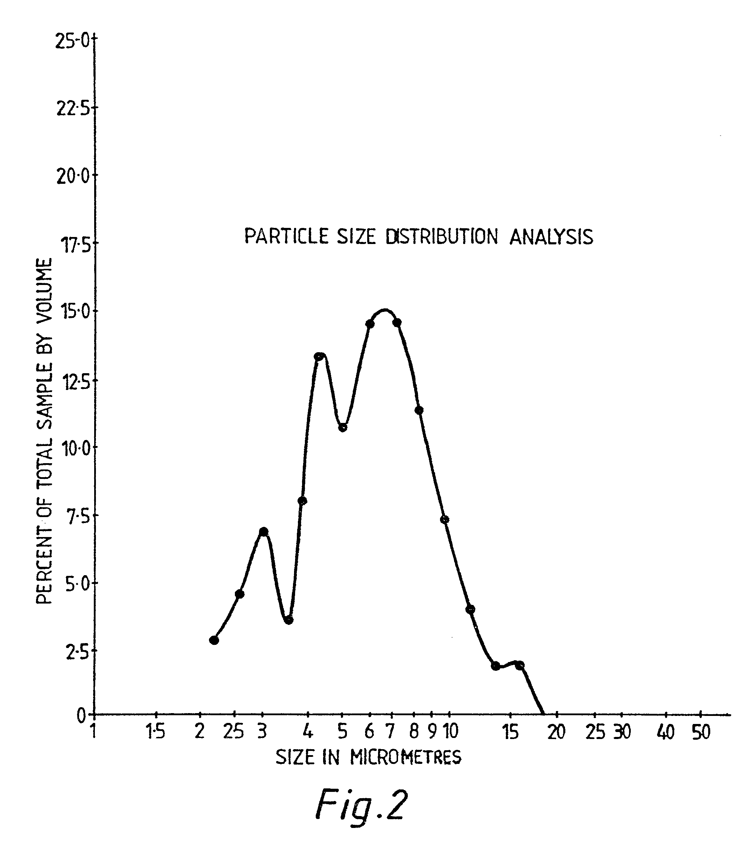

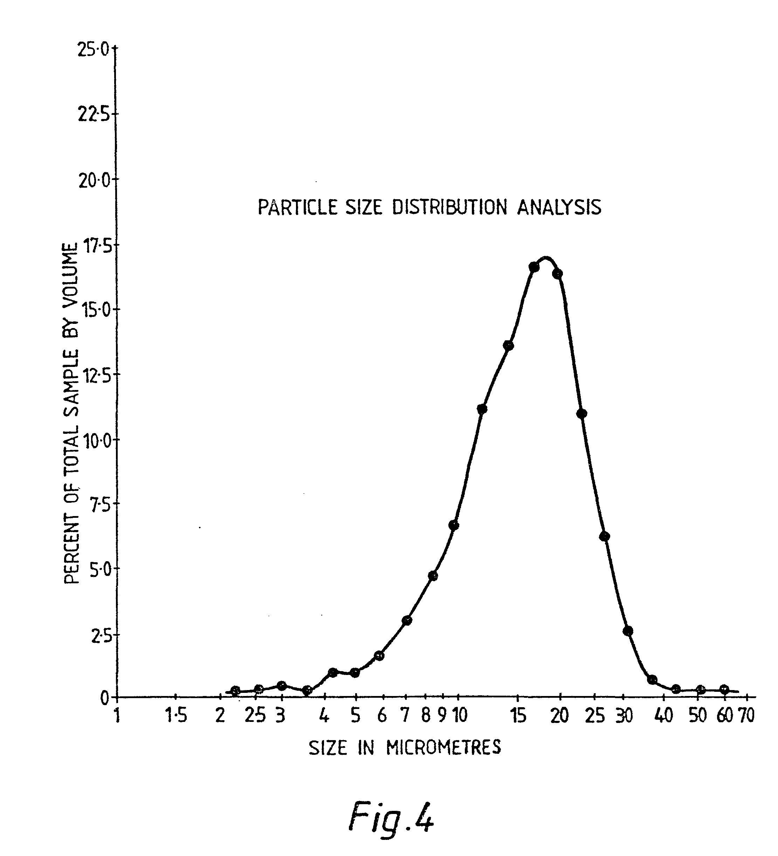

Fig. 2 and 4 show the particle size distribution analyses of the lithium carbonate material shown in Fig. 1 and 3. In each case the analysis is taken on readings given by a HIAC (Trade Mark) Particle Size Analyser (PA-720) using as carrier fluid a 0.5% $^w/_w$ solution of Span 20 (a non-ionic surfactant, "Span" being a Trade Mark) in toluene. It can be seen that the distribution curve of Fig. 2 shows a number of defined peaks, whilst the particle size distribution of the lithium carbonate material of Fig. 4 is essentially monomodal.

As previously indicated, the lithium carbonate material of Fig. 3 and 4 gives much better results from the point of view of a satisfactory release pattern for therapeutic purposes of lithium than does the material of Fig. 1 and 2. Whilst we do not wish to be bound by any theory as to why this is so, we believe that the coating round the cluster of crystals of the material of Fig. 1 penetrates into the cluster and round, or nearly round individual particles thus to fill or partially fill the void spaces between the individual crystals comprising the cluster, with the result that, when the coating is exposed to the eroding action of fluid and movement in the gastro-intestinal tract, an insufficient proportion of the surface of the lithium carbonate is exposed and available for solution in the body fluids at any given point in time.

*Example:*

A batch was prepared with the following ingredients:

| | |
|---|---|
| Lithium Carbonate Ph. Eur | 60 kg |
| Precirol ® | 5.85 kg |
| Mannitol B.P. | 9.9 kg |
| Acacia powder B.P. | 3 kg |
| Sodium Lauryl Sulphat B.P. | 318 g |
| Magnesium Stearate B.P. | 375 g |
| Maize Starch B.P. | 3.43 kg |

The lithium carbonate was supplied by Metallgesellschaft AG, and was in the form of acicular crystals with not more than 10% having a particle diameter above 30 μm. The particle size distribution was found to be monomodal, the particle size distribution analysis being similar to that shown in Fig. 4.

"Precirol" (Trade Mark) is a mixture of glyceryl mono-, di- and tri-esters of palmitostearic acids, principally palmitic and stearic acids, supplied by Messrs. Gattefosse of France.

The lithium carbonate was blended to ensure uniformity.

The mannitol was sieved through a 600 μm aperture screen and mixed in a mixer with the lithium carbonate and all the other ingredients except the Precirol ®. The mixer had a jacket for heating by steam or hot water and possessed a horizontal mixer shaft bearing 4 Z-shaped blades. The temperature was measured by means of a metal encased thermometer dipping at least 6 inches into the powder. Mixing and controlled heating were continued until a uniform temperature of 70° C was attained and held for 15 min.

An alcoholic solution of Precirol ® was prepared by dissolving 5.85 kg in 24 l of alcohol (industrial methylated spirit), and bringing the temperature of the solution to 72° C. This solution was poured onto the heated powdered mixture of the other ingredients and mixed for 15 min. Twelve litres of water were poured onto the mixture and the whole mass was kneaded.

The resulting mass was then dried at 40° C by evaporation, e.g. in an Aeromatic dryer. The crude granulate was then passed through a sieve with 1.40 mm apertures, and submitted to a sieve analysis with necessary adjustment made by adding particles from a previous batch to ensure a correct size distribution, and particularly that between 70 and 80% are retained on a sieve of 250 μm aperture size, while the remainder pass through such a sieve.

The resulting mixture was then compressed into tablets on a rotary tabletting machine with concave tipped rod-shaped punches. The resulting tablets had a weight of about 550 milligrams, a Monsanto hardness of 4 and contained about 400 milligrams of lithium carbonate and 39 milligrams of Precirol ®.

**Claims**

1. A sustained release lithium carbonate tablet containing particles of lithium carbonate coated with a sustained release agent, characterised in that the lithium carbonate material comprises acicular crystals with an essentially monomodal particle size distribution in which the curve showing volume percentages of particles of different sizes shows a peak at a particle diameter of between 10 and 25 μm, not more than 20% by volume of the material having a particle diameter above 30 μm.

2. A sustained release lithium carbonate tablet according to Claim 1, wherein the sustained release agent comprises a mixture of glyceryl mono-, di- and tri-esters of one or more straight chain saturated fatty acids.

3. A sustained release lithium carbonate tablet according to Claim 1 or Claim 2, wherein not more than 10% of the lithium carbonate material has a particle diameter above 30 μm.

4. A method of making tablets according to Claim 1, which comprises the steps of—

(*a*) mixing (*i*) acicular crystals of lithium carbonate having an essentially monomodal particle size distribution in which the curve showing volume percentages of particles of different sizes shows a peak at a particle diameter of between 10 and 25 μm, not more than 20% by volume of the material having a particle diameter above 30 µm; (*ii*) a sustained release agent comprising a mixture of glyceryl mono-, di- and tri-esters of one or more straight chain saturated fatty acids; and (*iii*) other innocuous compounding materials;

(*b*) granulation of the said mixture by a moist granulation technique;

(*c*) screening to form a granulate of appropriate grain sizes; and

(*d*) compacting the granulate into tablets.

5. A method according to Claim 4, wherein the screening is to give a granulate whose particles all pass through a sieve with 1.40 mm apertures, where 20 to 30% pass through a sieve with 250 µm apertures while 70 to 80% are retained on a sieve with 250 µm apertures.

6. A method according to Claim 4 or Claim 5, wherein not more than 10% of the lithium carbonate material has a particle diameter above 30 µm.

## Patentansprüche

1. Eine Lithiumcarbonattablette mit verzögerter Wirkstoffabgabe, die Lithiumcarbonatteilchen enthält, die mit einem die Wirkstoffabgabe verzögernden Mittel beschichtet bzw. überzogen sind, dadurch gekennzeichnet, dass das Lithiumcarbonatmaterial nadelförmige Kristalle mit einer im wesentlichen monomodalen Korngrössenverteilung aufweist, worin die Kurve, welche die Volumprozentsätze der Teilchen von verschiedenen Grössen darstellt, eine Spitze bei einem Teilchendurchmesser von 10-25 µm zeigt, wobei nicht mehr als 20 Vol.-% des Materials einen Teilchendurchmesser von mehr als 30 µm aufweisen.

2. Eine Lithiumcarbonattablette mit verzögerter Wirkstoffabgabe nach Anspruch 1, worin das die Wirkstoffabgabe verzögernde Mittel eine Mischung aus Glyceryl-mono-, -di- und -triestern einer oder mehrerer geradkettigen, gesättigten Fettsäure n aufweist.

3. Eine Lithiumcarbonattablette mit verzögerter Wirkstoffabgabe nach Anspruch 1 oder 2, worin nicht mehr als 10% des Lithiumcarbonatmaterials einen Teilchendurchmesser von mehr als 30 µm aufweisen.

4. Ein Verfahren zur Herstellung von Tabletten nach Anspruch 1, umfassend die Schritte:

(a) Mischen von (i) nadelförmigen Kristallen von Lithiumcarbonat mit einer im wesentlichen monomodalen Korngrössenverteilung, worin die Kurve, die die Volumprozentsätze von Teilchen von verschiedenen Grössen darstellt, eine Spitze bei einem Teilchendurchmesser von 10-25 µm aufweist, wobei nicht mehr als 20 Vol.-% des Materials einen Teilchendurchmesser von mehr als 30 µm besitzen; (ii) einem die Wirkstoffabgabe verzögernden Mittel, das ein Gemisch aus Glyceryl-mono-, -di- und -triestern einer oder mehrerer geradkettigen, gesättigten Fettsäure n umfasst und (iii) anderen verträglichen Mischungsbestandteilen bzw. Formulierungshilfen;

(b) Granulation des genannten Gemisches nach einem feuchten Granulationsverfahren;

(c) Sieben, um ein Granulat von entsprechenden Teilchengrössen zu bilden, und

(d) Verpressen des Granulates zu Tabletten.

5. Ein Verfahren nach Anspruch 4, worin das Sieben ein Granulat ergibt, dessen Teilchen alle durch ein Sieb mit einer Maschenweite von 1,40 mm fallen, wobei 20-30% durch ein Sieb mit einer Maschenweite von 250 µm fallen, während 70-80% der Teilchen auf einem Sieb mit einer Maschenweite von 250 µm zurückgehalten werden.

6. Ein Verfahren nach Anspruch 4 oder 5, worin nicht mehr als 10% des Lithiumcarbonatmaterials einen Teilchendurchmesser von mehr als 30 µm aufweisen.

## Revendications

1. Tablette de carbonate de lithium à mise en liberté soutenue, contenant des particules de carbonate de lithium enduites d'un agent de mise en liberté soutenue, caractérisée en ce que la matière carbonate de lithium comprend des cristaux aciculaires ayant une distribution de dimensions de particule essentiellement monomodale dans laquelle la courbe indiquant les pourcentages en volume des particules des différentes dimensions montre un pic à un diamètre de particule entre 10 et 25 µm, 20% en volume de la matière au plus ayant un diamètre de particule supérieur à 30 µm.

2. Tablette de carbonate de lithium à mise en liberté soutenue selon la revendication 1, caractérisée en ce que l'agent de mise en liberté soutenue comprend un mélange de mono-, di- et tri-estèrs glycéryliques d'un ou plusieurs acides gras saturés à chaîne droite.

3. Tablette de carbonate de lithium à mise en liberté soutenue selon la revendication 1 ou la revendication 2, dans laquelle au plus 10% de la matière carbonate de lithium a un diamètre de particule supérieur à 30 µm.

4. Procédé de fabrication de tablettes selon la revendication 1, comprenant les stades:

(a) de mélange de cristaux aciculaires de carbonate de lithium (i) ayant une distribution des dimensions de particule essentiellement monomodale dans laquelle la courbe montrant les pourcentages en volume des particules de différentes dimensions montre un pic à un diamètre de particule entre 10 et 25 µm, au plus 20% en volume de la matière ayant un diamètre de particule supérieur à 30 µm, d'un agent de mise en liberté soutenue (ii) comprenant un mélange de mono-, di- et tri-esters glycéryliques d'un ou plusieurs acides gras saturés à chaîne droite et (iii) d'autres matières de compoundage inoffensives,

(b) de granulation de ce mélange par une technique de granulation au mouillé,

(c) de tamisage pour former un granulat ayant des dimensions de grain appropriées, et

(d) de compactage du granulat en tablettes.

5. Procédé selon la revendication 4, caractérisé en ce que le tamisage a pour but de donner un granulat dont les particules passent toutes à travers un tamis ayant des ouvertures de 1,40 mm, où 20 à 30% passent à travers un tamis ayant des ouvertures de 250 μm tandis que 70 à 80% sont retenues sur un tamis ayant des ouvertures de 250 μm.

6. Procédé selon la revendication 4 ou la revendication 5, dans lequel au plus 10% de la matière carbonate de lithium a un diamètre de particule supérieur à 30 μm.

# Fig.1

0·1 mm

PARTICLE SIZE DISTRIBUTION ANALYSIS

*Fig.2*

# Fig.3

0·1mm

PARTICLE SIZE DISTRIBUTION ANALYSIS

Fig.4